# EUROPEAN PATENT SPECIFICATION

(11) **EP 1 980 198 B1**
(45) Date of publication and mention of the grant of the patent: **07.12.2016**
(21) Application number: 08006969.3
(22) Date of filing: 08.04.2008
(51) Int. Cl.: A61B 3/10, A61B 5/00, A61B 10/00, G01N 33/52, G01N 33/487

(54) **Tear meniscus test device and its manufacturing method**
Meniskusreißtestvorrichtung und Verfahren zu seiner Herstellung
Dispositif de test des lésions méniscales et son procédé de fabrication

(30) Priority: 10.04.2007 JP 2007103224
(43) Date of publication of application: 15.10.2008
(73) Proprietor: Echo Electricity Co., Ltd., Kawamata-machi Date-gun Fukushima 960-1407 (JP)
(72) Inventor: Ishida, Katsushi, Tokyo (JP); Tsubota, Kazuo, Chiba-ken (JP)
(74) Representative: Wolff, Felix

(56) References cited:
- EP-A- 0 391 109
- EP-A- 0 426 307
- EP-A- 1 692 999
- WO-A-96/21508
- WO-A1-00/20626
- DE-A1- 4 335 961
- JP-A- 2005 253 700
- US-A- 4 334 879
- US-A- 4 761 381
- US-A- 5 170 799
- US-A- 5 260 031
- US-A1- 2007 048 224

## Description

### BACKGROUND OF THE INVENTION AND RELATED ART STATEMENT

### 1. FIELD OF THE INVENTION

The present invention relates to a tear meniscus test device and a manufacturing method therefor.

### 2. DESCRIPTION OF RELATED ART

Conventionally, the Schirmer's test is known as a method for measuring the amount of tears in the eye. In this test, one end of a filter paper strip having a width of about 5 mm is placed between the inner edge of the lower eyelid and the eyeball, so that tears are absorbed into the paper strip and the penetration length of the tears is subsequently measured. When the Schirmcr's test paper is used, pain is involved because the eyelid presses the test paper against the cornea. Thus, an eye-drop anesthetic must be used. Also, the irritation caused by the contact of the Schirmer's test paper with the lower eyelid may increase the tear secretion, thus making it difficult to perform exact measurement. Moreover, long measurement time, typically 5 minutes, is required, thus placing considerable burden on the patient. In some cases, the eye surface may be damaged.

As an alternative to the Schirmer's test, a method that uses a sterile cotton thread in place of the Schimer's test paper is also employed. In this method as well, some pain is involved since one end of the cotton thread is placed between the lower eyelid and the eyeball as in the Schirmer's test. Also, the rate at which the cotton thread absorbs tears is restricted by an absorption rate defined by material properties inherent to the cotton thread. It is, therefore, difficult to reduce the measurement time beyond the inherent absorption rate of the cotton thread. Also, the cotton thread expands and contracts easily, and a scale for measuring a wetted portion is not marked on the cotton thread itself, so that the length must be read with a separate graduated ruler, this work being troublesome.

For the tear meniscus test, not only the amount of ooze but also the quality of test must be considered. As the test method, so-called tear clearance test is available. In this method, a drop of 5% fluorescein pigment solution is introduced into the conjunctival sac for each eye and, after 5 minutes, the Schirmer's test paper is used to check the dilution rate. As another test method, the measurement of so-called tear film break-up time (BUT) is available. In this method, tears are stained with fluorescein pigment and the time period taken for the tears to dry while the eye is kept open is measured. However, particularly for a patient with reduced tear secretion, the pigment-based methods, such as the clearance test and the tear film break-up time (BUT) measurement, may cause an adverse effect on the cornea because the pigment remains on it as a foreign matter.

In recent years, attention has been directed to the relationship between medical symptoms and the amount of tears present in what is called a tear meniscus formed between the lower eyelid and the eyeball, and the importance of measuring the amount of tears in this tear meniscus is increasingly recognized. However, the amount of tears in the meniscus is small, at most about several microliters, thus posing a problem in that it is difficult to perform measurement.

With any test method using currently-available devices, which is often expensive, for a doctor to perform diagnosis, he or she needs to perform measurement while observing the tears and the eye surface by using a slit (i.e., a slit lamp microscope). That is, a test device that can readily perform on-site manual quantitative measurement on a tear meniscus has not been available.

Under such circumstances, a test device for quantitatively measuring the amount of tears on a tear meniscus has been invented, and has been disclosed in Patent Document 1 (Japanese Unexamined Patent Application Publication No. 2005-253700) by an applicant who is the same as the applicant of the present invention.

Document EP1692999 A discloses a tear meniscus test device comprising: a body part having an elongated shape, wherein the body part comprises a synthetic resin material or a synthetic rubber material; a groove is formed in the body part along a length direction of the body part; and the body part is flexible and the front end part thereof is rounded; and wherein there is a transparent film bonded above the body part and the document discloses injection molding of the device.

Document WO0020626 A1 discloses a manufacturing method for a test device for bodily fluids, comprising the steps of indenting, embossing or otherwise deforming a sorbent material with a base material, or depositing a sorbent material after a base material is indented, embossed, or otherwise deformed to make a recess.

### OBJECT AND SUMMARY OF THE INVENTION

As described above, Patent Document 1 discloses a test device for quantitatively measuring the amount of tears in a tear meniscus. However, a further improved test device has been demanded.

To solve the above problem, the tear meniscus test device in accordance with the present disclosure includes a body part having an elongated shape, which is formed of a synthetic resin material or a synthetic rubber material formed with a groove along the length direction thereof; and a water absorbing member arranged in the groove**.**

The water absorbing member can be formed of a hydrophilic material. As a specific material, vegetable fibers, animal fibers, and synthetic resins such as rayon, acetate, nitrocellulose, polyethersulfone, polysulfone, nylon, polypropylene, vinyl, acrylic copolymers, and cellulose polyvinylidene difluoride (PVDF), having a hydrophilic property can be cited. To make the material hydrophilic, treatment using a surface-active agent may be performed, or a hydrophilic group such as amino group, hydroxyl group, and carboxyl group may be chemically modified on the base material.

As the water absorbing member, various types of hydrophilic filter papers or membranes can be used to measure the amount of tears. For example, a commercially available filtration membrane or chromatography membrane is preferable because it is flexible, causes less irritation to the eye, and has a high wetting rate and a constant wetting rate and quantitativeness.

Also, to easily determine the amount of absorbed tears, a pigment may be placed in a dot pattern or continuously over a part or the whole of the surface of the water absorbing member.

To facilitate coloring onto the back surface of the water absorbing member or to facilitate handling, the back surface of the water absorbing member can be reinforced by a thin film. In the case where the aforementioned various types of membranes are used, the membrane is a continuous porous body that has a thin-film shape and a minute pore diameter so as to assist absorption and diffusion, and voids occupy most of the volume of the membrane, so that the membrane is easily broken. Therefore, by backing the membrane with a thin resin film, the membrane is reinforced and made easy to handle, and further by coloring this film, the back surface of the water absorbing member can be colored. The back surface of the water absorbing member means the surface facing to the bottom part of the groove.

As the member constituting the body part, a synthetic resin having flexibility and a low hygroscopic and water absorbing property is preferably used. For example, vinyl, polypropylene, polyethylene, nylon, polyisoprene, silicone rubber, and urethane rubber are suitable. Also, white color is preferable because it has a high contrast and is visible easily.

Further, the front end part of the tear meniscus test device, which is brought into contact with the eye of a patient, preferably has a round shape (a shape having no corner, such as a semicircular shape or an elliptical shape) so as not to give unnecessary irritation to the eye. Also, the groove may be continuous from the front end of the tear meniscus test device to the rear end thereof so as to form an opening at the front end or may be terminated at an intermediate position so that a gripping portion of test device is allowed to remain.

The dimensions of the tear meniscus test device are preferably 1 to 5 mm in width, not thicker than 1 mm in thickness, and 5 to 70, mm in length so that the test device can easily be held by hand. Also, a scale is preferably marked on the body part along the groove. Also, to distinguish the device for the right eye and the device for the left eye from each other, some identification measures may be taken: for example, a letter is printed at the rear end of the tear meniscus test device, or the cut position is changed.

The manufacturing method for a tear meniscus test device in accordance with the present invention can include the steps of forming a body part sheet by forming elongated slits by stamping a sheet formed of a synthetic resin or synthetic rubber and by bonding an adhesive film to one surface of the sheet; cutting a water absorbing member sheet into an elongated shape; arranging a water absorbing member, which is obtained by cutting the water absorbing member sheet into an elongated shape, in a groove formed by the slit and bonding the water absorbing member to the adhesive film forming the bottom part of the groove; and cutting the body part sheet into an elongated shape along the groove.

One surface of the water absorbing member sheet may be covered with a reinforcing film, and the water absorbing member cut into an elongated shape may also be embedded in the groove so that the reinforcing film is in contact with the adhesive film, and also the manufacturing method can include a step of coloring the reinforcing film.

Also, the manufacturing method for a tear meniscus test device in accordance with the present invention can include the steps of forming an elongated body part sheet formed with a concave groove along the length direction thereof by extrusion molding or injection molding a synthetic resin or synthetic rubber; cutting a water absorbing member sheet provided with an adhesive film on one surface thereof into elongated strips; and arranging a water absorbing member, which is obtained by cutting the water absorbing member sheet into an elongated shape, in the groove in the body part sheet and bonding the water absorbing member to the bottom part of the groove via the adhesive film.

The one surface of the water absorbing member sheet may be covered with a reinforcing film, and the adhesive film may be bonded to the surface of the reinforcing film, and also the manufacturing method can include a step of coloring the reinforcing film.

For the tear meniscus test device in accordance with the present invention, since the water absorbing member is arranged in the groove formed in the body part formed of a material having a low hygroscopic and water absorbing property, the tears do not diffuse into the body part. Therefore, when one end part of the water absorbing member is brought into contact with the patient and the tears are absorbed by the water absorbing member, the tear penetration concentrates on the other end part. Therefore, even if the amount of tears is small, the measurement can be performed in a short period of time, and the amount of tears can be determined more exactly.

### BRIEF DESCRIPTION OF THE DRAWINGS

FIG 1 is a schematic plan view of a tear meniscus test device in accordance with a first embodiment of the present invention;
FIG 2 is an exploded perspective view of a tear meniscus test device in accordance with a first embodiment of the present invention;
FIG. 3 is a sectional view showing one example of a cross section taken along the line A-A of a tear meniscus test device in accordance with a first embodiment of the present invention;
FIG. 4 is a sectional view showing another example of a cross section taken along the line A-A of a tear meniscus test device in accordance with a first embodiment of the present invention;
FIG. 5 is a schematic plan view of a tear meniscus test device in accordance with a second embodiment of the present invention;
FIG 6 is a schematic plan view of a tear meniscus test device in accordance with a third embodiment of the present invention;
FIG 7 is a schematic plan view of a tear meniscus test device in accordance with a fourth embodiment of the present invention;
FIG 8 is a schematic view showing a manufacturing method in accordance with one embodiment of the present invention; and
FIG. 9 is a perspective view showing a state of a tear meniscus.

### DETAILED DESCRIPTION OF THE PREFERRED EMBODIMENTS

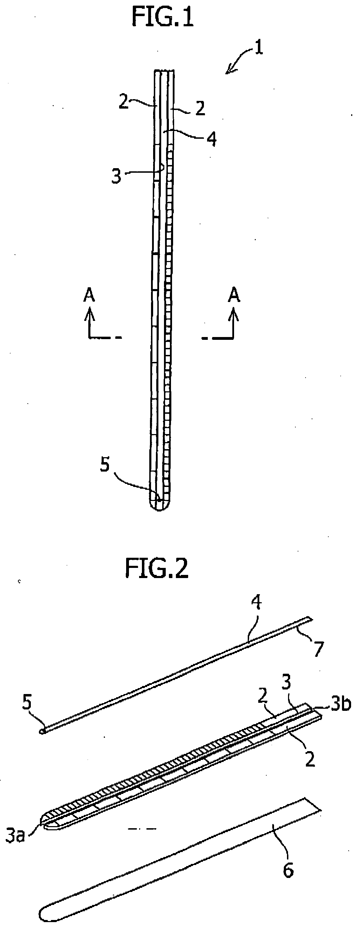

A first embodiment of the present invention will now be described by reference to the accompanying drawings. FIGS. 1 and 2 show a tear meniscus test device in accordance with the first embodiment of the present invention. FIG 1 is a view of the tear meniscus test device viewed form the front, and FIG 2 is an exploded perspective view of the tear meniscus test device.

A tear meniscus test device 1 has an elongated shape and flexibility, and is provided with a body part 2 formed of a material having a low hygroscopic and water absorbing property. The body part 2 is formed with a groove 3 along the length direction thereof. This groove 3 has open end parts 3a and 3b in one end part (hereinafter referred to as a front end part) in which the tear meniscus test device 1 is brought into contact with a patient and in the other end part (hereinafter referred to as a rear end part), respectively. On the surface of the body part 2, a scale can be printed along the groove 3.

In the groove 3, a water absorbing member 4 that is brought into contact with the lower eyelid of the patient to absorb the tear is embedded. The length and width of the water absorbing member 4 arc set so as to be almost equal to those of the groove 3. The thickness of the water absorbing member 4 is set so as to be almost equal or smaller than the depth of the groove 3 when the water absorbing member 4 is arranged in the groove 3 to lessen the decrease in groove effect. In the front open end part 3a, the water absorbing member 4 embedded in the groove 3 is exposed in the front end direction, and forms a part of the front end part of the tear meniscus test device 1.

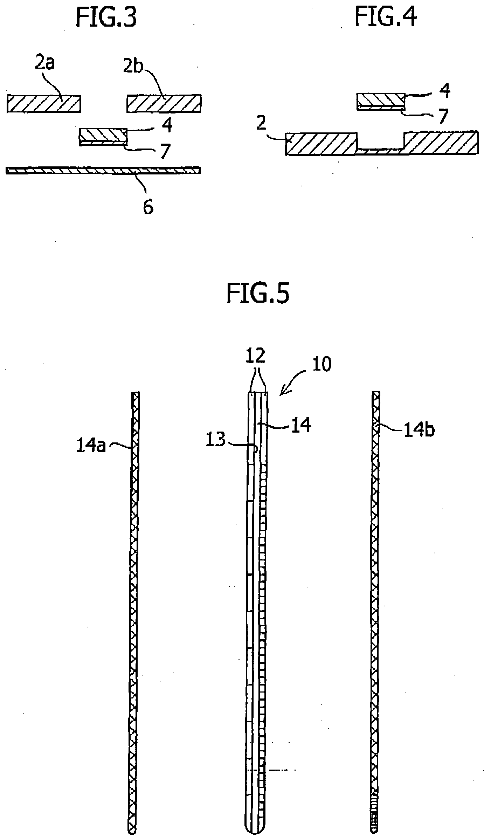

FIG 3 shows a cross section of the test device 1 cut along the line A-A of FIG 1. In the mode shown in FIG 3, the water absorbing member 4 has a reinforcing film 7 on the back surface thereof, so that the water absorbing member 4 is fixed to a fixing adhesive film 6 via the reinforcing film 7 together with body side parts 2a and 2b. Instead of the use of the fixing adhesive film 6, any of an adhesive, a gluing agent, a heat seal, and the like may be used to bond the water absorbing member 4 and the body side parts 2a and 2b to a fixing film 6. Also, as shown in FIG 4, it is possible to form the body part 2 having the groove 3 by extrusion molding or injection molding and to bond the water absorbing member 4 to the bottom part of the groove 3 via the reinforcing film 7.

The front end part of the tear meniscus test device 1 is fabricated into a semicircular shape. In the front end part of the water absorbing member 4, a water-soluble pigment 5 can be placed at a location slightly distant from the front end. As the water-soluble pigment 5, a pigment such as Blue No.1 or Cape jasmine blue pigment of natural pigment can be used.

A mark (not shown) indicating right eye or left eye can be placed in the rear end part of the tear meniscus test device 1. By placing the mark indicating right eye or left eye, the test can be performed without of a hitch, and a mistake that only one eye is tested two times can be prevented. In the above-described embodiment, the groove 3 is formed so as to have a length almost equal to that of the body part 2, and is configured so as to have the open end part 3b in the rear end part of the body part 2. However, the groove 3 may be terminated in front of the rear end part of the body part 2, and the water absorbing member 4 having a length almost equal to that of this groove 3 may be arranged in the groove 3. In this case, in the body part 2, the mark indicating right eye or left eye can be printed in a region ranging from the position at which the groove 3 is terminated to the rear end part of the body part 2.

When the front end part of the tear meniscus test device 1 is brought into contact with the lower eyelid of the patient, the tears of the patient are first absorbed by the water absorbing member 4 and dissolve the added water-soluble pigment 5, and then penetrate in the direction toward the rear end part of the tear meniscus test device 1 along the water absorbing member 4 held between the side walls of the groove 3. The tears penetrate together with the dissolved pigment, and this penetration continues as long as the tears are supplied from the eye. The penetration length of tears can easily be observed visually by the pigment, and can also be read from the scale on the body part 2.

Next, the method for using the tear meniscus test device 1 is explained.

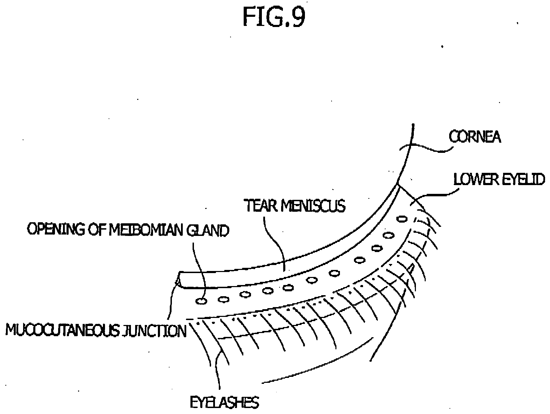

FIG 9 shows a state in which a tear meniscus (also referred to as a "tear triangle") is formed along an interface between the cornea and the lower eyelid. In the tears forming the tear meniscus, a negative pressure is generated that is proportional to the surface tension of tears and is inversely proportional to its radius of curvature, so that a concave surface called a mucocutaneous junction is formed.

A mucous layer containing a large amount of polysaccharides, such as mucin, lies on the cornea. On top of the mucous layer, a tear aqueous layer and a lipid layer are formed. The tear aqueous layer connects therebelow with the tear meniscus. The amount of tears in the tear meniscus is related to the medical conditions of dry eye, but it is so small and difficult to measure. However, for the tear meniscus test device 1 in accordance with the present invention, placing the front end of the test device at such a position that the front end thereof is in contact with the tear meniscus on the upper end of the lower eyelid allows measurement in a period of time as short as about 5 seconds. In this case, since the front end of the test device need not be in contact with the cornea, irritation of the eye is also reduced: Additionally, since the test can be performed in such a short period of time, the test device 1 has only to be held by hand or by using a simple supporter device.

An experiment described below was conducted. The tear meniscus of a subject was stained with fluorescein and the test device of the present invention was applied to the tear meniscus for 5 seconds. Then, the eye of the subject was exposed to light. Resultantly, light emission from the fluorescein stain solution was not observed. This experimental result is the basis of the fact that the tear meniscus test device 1 in accordance with the present invention can measure the amount of tears in a period of time as short as about 5 seconds.

For the tear meniscus test device 1, since the water absorbing member 4 is arranged in the groove 3 formed in the body part 2 having a low hygroscopic and water absorbing property, the wetting rate increases remarkably as compared with the case where the water absorbing member 4 is exposed without being embedded in the groove 3. Also, since the body part 2 formed with the groove 3 is formed of a material having a low hygroscopic and water absorbing property, the tears absorbed do not diffuse into the body part 2. Therefore, the tears penetrate concentratedly toward the rear end part of the tear meniscus test device 1. For this reason, even if the amount of tears is minute, the measurement can be performed in a short period of time, and the amount of tears can be determined more exactly.

By using the tear meniscus test device 1, for example, a doctor can quantitatively measure the amount of tears in the tear meniscus easily in a short period of time (about 5 seconds), and can test tear secretion while observing the tears and the eye surface by using a slit lamp microscope. The burden such as pain on the patient is reduced because the measurement time is short, and damage to the eye surface, which has been described in the conventional example, is avoided. Therefore, a tear meniscus test device (or strip tear meniscometry) for easily measuring the tear meniscus of the patient is realized.

In addition, since the body part 2 is flexible, the pain of the patient can be reduced. Also, the flexibility and strength of the tear meniscus test device can be enhanced by the body part 2 that holds the water absorbing member 4 therebetween. Therefore, the tear meniscus test device has an effect of being easy to use without being broken or bent extremely at the time of tear test.

Further, since on the surface of the body part 2, the scale is marked along the groove 3, the length of a portion wetted by tears can be read easily. Also, since the mark indicating right eye or left eye is placed in the rear end part of the tear meniscus test device 1, it can easily be judged which of the right and left eyes was tested.

Hereunder, second to fourth embodiments of the present invention are explained. The basic configurations of the tear meniscus test devices and the methods for using the tear meniscus test devices in the second to fourth embodiments are similar to those in the first embodiment, but the method for adding pigment or coloring is different.

FIG 5 shows a tear meniscus test device 10 in accordance with the second embodiment of the present invention. Like the first embodiment, the tear meniscus test device 10 has an elongated shape and flexibility, and is provided with a body part 12 formed of a material having a low hygroscopic and water absorbing property The body part 12 is formed with a groove 13 along the length direction thereof, and a water absorbing member 14 is embedded in the groove 13. However, unlike the first embodiment, the pigment 5 is not added. Instead of the addition of pigment, the reinforcing film of the water absorbing member 14 is colored. In the state in which the water absorbing member 14 is dry, the color put on the reinforcing film is invisible from the front of the tear meniscus test device 10. When the water absorbing member 14 is wetted by tears, the degree of light transmission in the wetted portion becomes high, so that the color put on the reinforcing film on the back surface of the water absorbing member 14 becomes visible. Therefore, from the length of the appearing colored portion, the length of tear penetration can be determined.

In FIG 5, the colored surfaces (colored surfaces of the reinforcing films) of two kinds of water absorbing members 14a and 14b, which are colored in different coloring patterns, are shown at the left and right of the tear meniscus test device 10, respectively. The reinforcing film of the water absorbing members 14a is colored with one color, a relatively dark color, so that at the time of measurement, the length wetted by tears is determined by one color. The reinforcing film of the water absorbing members 14b is colored in a pattern in which a parameter reflecting the diagnosis standard is shown by a plurality of colors, so that the degree of dry eye can be indicated by the color at the front end of the portion wetted by tears without reading the scale.

In the second embodiment, the reinforcing film is colored, and pigment is not added to the water absorbing member directly, so that the coloring of the water absorbing member due to the water-soluble pigment that has been used in the first embodiment does not occur. Therefore, in addition to the effect achieved by the tear meniscus test device 1 in accordance with the first embodiment, at the time of qualitative test of tears, the turbidity with a test pigment, for example, a fluorescein or triphenylmethane-based pigment is hindered, and the clearance test and the qualitative and quantitative measurement of protein can be performed. In such colorimctric methods, the judgment can be made more easily after the water absorbing member has dried off.

The protein in tears reflects the qualitative property of tears. Especially for a contact lens wearer, the property of protein is an important factor as one of the selection standards of lens material. If much protein is contained in the tears, the tears arc liable to adhere to the contact lens, which not only causes contamination but also decreases the oxygen permeability. This also results in the deterioration of lens wearing sense, the weakening of eyesight, and the disorder of the cornea. As a method for testing protein in tears, the protein error method as disclosed in Japanese Unexamined Patent Application Publication No. 2004-347481 is available. In this method, after the tears have been put into a predetermined tear collecting medium, the medium is caused to react with a triphenylmethane-based pigment to detect the content of protein in the tears in color development under an acidic condition.

In the tear meniscus test device 10, for example, a nitrocellulose can be used as a material of the water absorbing member. The nitrocellulose has a functional group having excellent protein adsorbing power. By using the nitrocellulose membrane, the protein in tears can be adsorbed intensely and captured (as one example, protein adsorbing power ≒ 30 [µg IgG/cm²]). Therefore, after the amount of tears has been measured by using the tear meniscus test device 10, the colorimetric analysis method and optical analysis method using a triphenylmethane-based pigment can be used as a simple test method for adsorbed protein, so that the qualitative or quantitative measurement of protein having high sensitivity can be performed.

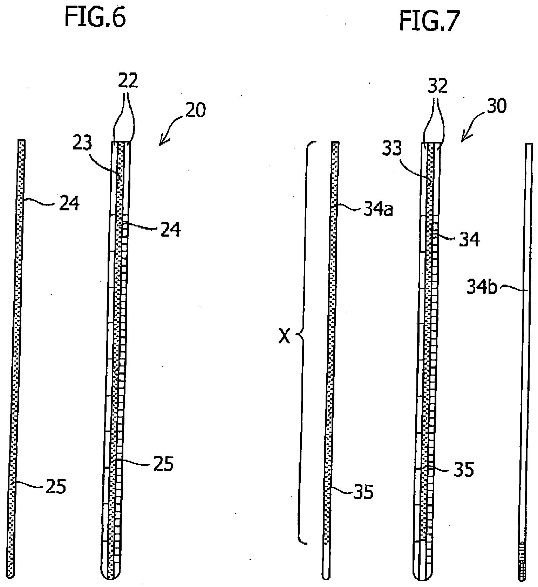

FIG 6 shows a tear meniscus test device 20 in accordance with the third embodiment of the present invention. Like the first embodiment, the tear meniscus test device 20 has an elongated shape and flexibility, and is provided with a body part 22 formed of a material having a low hygroscopic and water absorbing property. The body part 22 is formed with a groove 23 along the length direction thereof, and a water absorbing member 24 is embedded in the groove 23. In this embodiment, however, unlike the first embodiment, the whole surface of the water absorbing member 24 is studded evenly with dot-shaped water-soluble pigments 25. As the water-soluble pigment 25, Blue No.1 or Cape jasmine blue pigment of natural pigment can be used. Also, the water-soluble pigment 25 can be printed in a dot shape by, for example, a commercially available inkjet printer.

The tears absorbed in the water absorbing member 24 dissolve the dot-shaped water-soluble pigments 25 interspersed evenly and move the pigments toward the rear end part along with the penetration of tears. Therefore, the end part of tear in the penetration direction is stained by the pigment, by which the length of tear penetration can be determined. In the case where the dot-shaped water-soluble pigments 25 are arranged densely, the whole surface of the water absorbing member 24 is in an all-over painted condition. Even in the case where the pigment is placed continuously over the water absorbing member 24, likewise, the dissolved pigment moves along with the penetration of tears, and the end part of tear in the penetration direction is stained, by which the length of tear penetration can be determined.

In this embodiment, the pigment is placed in a dot pattern or continuously over the whole surface of the water absorbing member 24. Therefore, in addition to the effect achieved by the tear meniscus test device 1 in accordance with the first embodiment, an advantage is offered that the penetration of tears can be seen with eyes distinctly even in the case where the front end of the water absorbing member 24 is searcely wetted because the amount of tears is extremely small or inversely in the case where a range reaching close to the limit of the test device 20 is wetted because the amount of tears is large. In the first embodiment, in the case where the amount of tears is extremely small, there is a possibility that the tears do not reach the pigment 5. In this case, the amount of tears is difficult to determine. Also, in the case where the amount of tears is large, when a fixed amount of pigment 5 is added, as the length of wetted portion increases, the pigment 5 is thinned, so that a problem in that the amount of tears is difficult to determine may occur on rare occasions. In this embodiment, the occurrence of such a problem can be reduced.

Also, the test device of this embodiment has another advantage as described below. In the first embodiment, the marked scale must be positioned with the position at which the pigment 5 is added being fixed. However, in this embodiment, since the dot-shaped water-soluble pigments 25 are interspersed evenly on the whole surface of the water absorbing member 24, the above-described positioning is not needed, which is advantageous in manufacturing. In the case where the pigment is placed in an over-all painted condition, the same advantage is offered.

FIG 7 shows a tear meniscus test device 30 in accordance with the fourth embodiment of the present invention. Like the first embodiment, the tear meniscus test device 30 has an elongated shape and flexibility, and is provided with a body part 32 formed of a material having a low hygroscopic and water absorbing property. The body part 32 is formed with a groove 33 along the length direction thereof, and a water absorbing member 34 is embedded in the groove 33. The water absorbing member 34 in the fourth embodiment has features of the water absorbing members in the second and third embodiments. In FIG 7, a top surface 34a and a back surface 34b of the water absorbing member 34 are shown at the left and right of the tear meniscus test device 30, respectively. On the top surface 34a, dot-shaped water-soluble pigments 35 are interspersed evenly in the region from a point at a specific distance from the front end part to the rear end part. The region in which the water-soluble pigments 35 arc interspersed is indicated by X on the surface 34a shown in FIG 7. On the back surface 34b, in the front end part, the reinforcing film is colored with a plurality of colors in a color-coded pattern. This color-coded pattern reflects the diagnosis standard of dry eye, and indicates a suspicious portion in which the possibility of dry eye is high. The region colored with the color-coded pattern corresponds to the back surface of the region in the front end part, in which the water-soluble pigments 35 are not interspersed, on the top surface 34a. By using the water absorbing member 34 configured as described above, a mode in which the amount of tears can be determined more easily without intermixture of colors is provided. Even in the case where the pigment is placed continuously over in place of the dot pattern, the same advantage is offered.

To clarify the advantages of the present invention, an experiment for confirming the groove effect in the present invention is explained. In this experiment, the wetting rate was compared between the tear meniscus test device 1 in which the water absorbing member 4 is arranged in the groove 3 formed of a material having a low hygroscopic and water absorbing property and only the water absorbing member that is not arranged in the groove 3. Here, the former is called a groove type, and the latter is called an open type. For the groove type, the tear meniscus test device 1 explained in the above-described first embodiment was used, and for the open type, a tear meniscus test device in which the water absorbing member 4 was exposed through a distance of about 25 mm from the front end part of the tear meniscus test device 1 by removing the fixing adhesive film and the body part 2 was used. The material forming the groove 3 in the tear meniscus test device 1 used in this experiment was urethane rubber, and as the water absorbing member, a nitrocellulose membrane was used.

First, a total of 10 samples of the groove type and the open type were affixed alternately to a double-faced adhesive tape with the positions of the front end parts thereof being aligned. The tape to which the samples had been affixed was immersed in a blue aqueous solution for 5 seconds in a wetting rate measuring instrument, and then was pulled up immediately to wipe off excess aqueous solution, by which the wetting rate was read. When the samples were affixed to the double-faced adhesive tape, for the samples of the groove type, the rear end side on which the tip end was not rounded was used as the front end part to prevent the centers of the samples from shifting. For each of the samples, the penetration length of the blue aqueous solution was measured, and the average value was calculated. As the result, the penetration length of the groove type was 9.8 mm on the average, and that of the open type was 6.9 mm on the average. Comparing both the average values with each other, the penetration length of the groove type was about 1.4 times that of the open type, that is, a high wetting rate was realized. As is also apparent from this experimental result, in the present invention, the absorbing ability of the water absorbing member is enhanced more by the arrangement of water absorbing member in the groove formed of a material having a low hygroscopic and water absorbing property.

Also, the fact that the nitrocellulose membrane is preferably used as the water absorbing member is also apparent from the experimental result of membrane wetting rate measurement explained below. Ten sheets of each of five kinds of membrane strips (1 cm x 4 cm) were immersed in the blue aqueous solution for 5 seconds in the wetting rate measuring instrument, and then was pulled up immediately to wipe off excess aqueous solution, by which the penetration length of the blue aqueous solution was measured.

As five kinds of membrane strips, the followings were used.
(1) AE100: Schleicher & Schucll, Inc., nitrocellulose, pore diameter 12 µm, thickness 100 µm
(2) CN140: Sartorius Ltd., nitrocellulose, pore diameter 8 µm, thickness 140 µm
(3) PR: Pall Corporation, polyethersulfone surface is modified by nitro group (trade name: Predator), thickness 140 µm
(4) SP5: Pall Corporation, hydrophilic polyethersulfone (trade name: Supore-5000D), pore diameter 5 µm, thickness 140 µm
(5) SP0.8: Pall Corporation, hydrophilic polyethersulfone (trade name: Supore-800), pore diameter 0.8 µm, thickness 140 µm

The above items (1) to (3) are immunochromatography membranes, and items (4) and (5) are precision filtration membranes.

The measurement results are given in the table below.

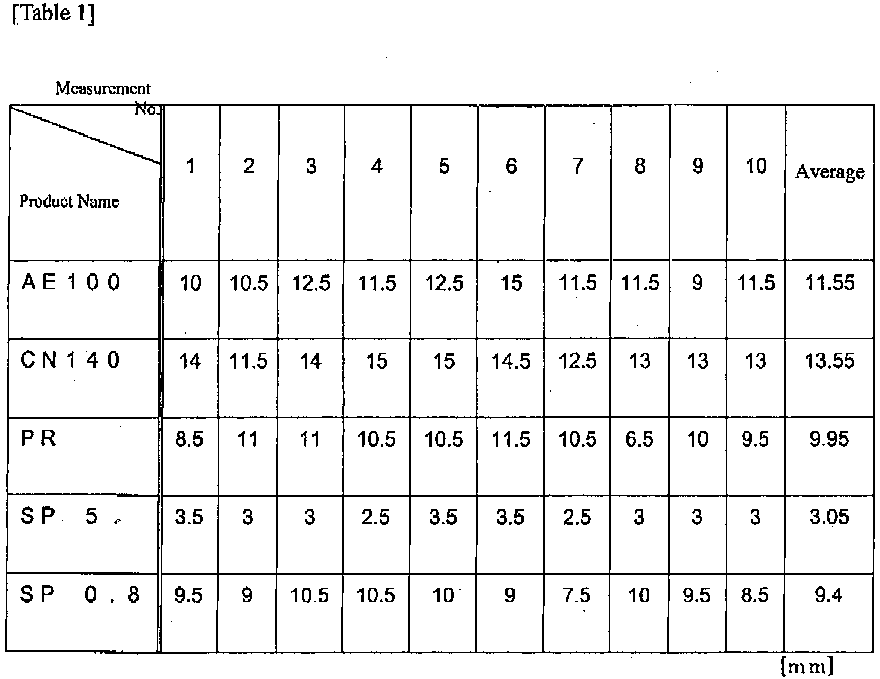

As given in Table 1, the nitrocellulose membranes of the items (1) and (2) have a large penetration length of the blue aqueous solution, that is, a high wetting rate as compared with other membranes. Therefore, the nitrocellulose membrane is preferably used as the water absorbing member.

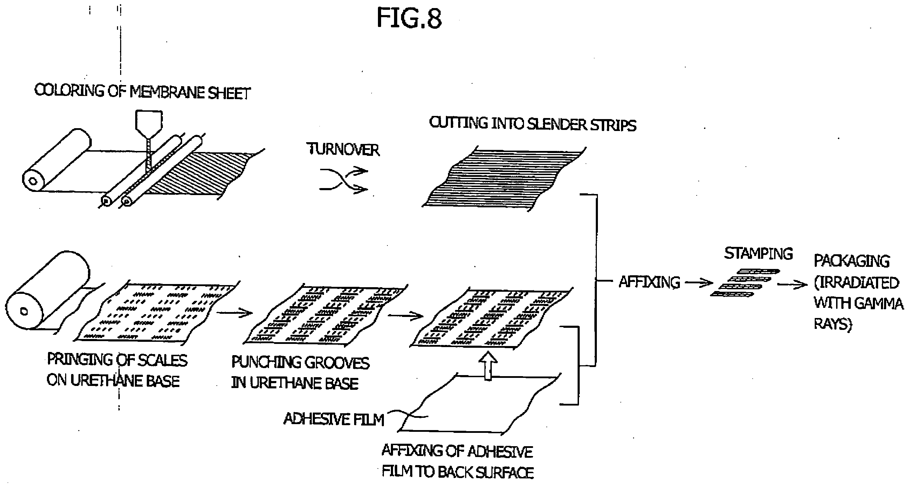

Next, a manufacturing method for the tear meniscus test device is explained by reference to FIG 8. Herein, the manufacturing method for the tear meniscus test device 10 using the water absorbing member 14a in the second embodiment is explained as an example.

To manufacture the water absorbing member 14a, first, a membrane sheet one surface of which is covered with a reinforcing film is colored by using a commercially available inkjet printer etc. At this time, the coloring is performed on the reinforcing film. In this embodiment, the reinforcing film before being colored is transparent. The colored membrane sheet is turned over by a turnover means, being arranged with the colored surface being directed downward, and is cut into elongated strips by a cutting means. The cut size is preset so that the length and width of the cut membrane are a length and width such that the cut membrane can be embedded in the groove 13 in the tear meniscus test device 10.

On the other hand, to manufacture the body part 12, on one surface of a urethane base sheet, scales are printed by using a printing means. Next, the grooves 13 are made, by using a grooving means, along the scales on the urethane base sheet on which the scales have been printed. To the back surface of the urethane base sheet in which the grooves 13 have been made (the surface on which the scales are not printed), a fixing adhesive film is affixed.

Next, the cut membranes, that is, the water absorbing members 14a arc arranged along the bottom parts of the grooves 13. At this time, the water absorbing member 14a is embedded in the groove 13 so that the colored reinforcing film surface comes into contact with the adhesive film forming the bottom part of the groove 13, and is fixed in the groove 13 by the adhesive film.

Next, the urethane base sheet in which the water absorbing members 14a have been fixed is stamped into an elongated shape having a rounded front end part, by which the tear meniscus test device 10 is manufactured. When the tear meniscus test device 10 is shipped as a product, the body strip is sterilized by the irradiation of gamma rays after being packaged.

Referring to FIG. 4, the tear meniscus test device in accordance with the present invention can be manufactured by forming the body part having the groove by extrusion molding or injection molding as described above.

In this case, a body part sheet is prepared by extrusion molding or injection molding a synthetic resin or synthetic rubber having a low hygroscopic and water absorbing property. This body part sheet is a sheet in which both of the body part for the right eye and the body part for the left eye are formed in a pair, and two grooves are formed along the length direction of the body part sheet for the right eye and the left eye. As shown in FIG 4, this groove does not penetrate in the thickness direction of the body part, and is formed as a concave part that is open upward in the thickness direction of the body part. Additionally, FIG 4 shows the sectional view of one tear meniscus test device for the right eye or the left eye. However, in the above-described body part sheet, since both of the body part for the right eye and the body part for the left eye are formed in a pair, in the cross-sectional shape thereof, two concave parts that are open upward in the thickness direction of the body part are formed. Then, a scale is printed, by a printing means, on the surface in which the grooves of the body part sheet arc formed.

On the other hand, for the water absorbing member, on a water absorbing member sheet one surface of which is covered with the reinforcing film, the adhesive film is bonded to the surface of the reinforcing film, and the water absorbing member sheet is cut into a plurality of elongated strips so that each of the strips can be arranged in the two grooves. The cut water absorbing member is arranged in each of the two grooves so that the surface on the adhesive film side of the cut water absorbing member sheet comes into contact with the bottom parts of the two grooves in the body part sheet, and the water absorbing member is fixed to the bottom part of the groove by the adhesive film.

Then, cut is made between the two grooves over the length direction to separate the body part for the right eye and the body part for the left eye from each other, by which the tear meniscus test device can be manufactured. Also, for the above-described water absorbing member, the adhesive film can also be bonded to the surface of the reinforcing film after the reinforcing film has been colored.

## Claims

1. A tear meniscus test device comprising:
(i) a body part having an elongated shape, wherein
- the body part comprises a synthetic resin material or a synthetic rubber material;
- a groove is formed in the body part along a length direction of the body part; and
- the body part is flexible and the front end part thereof is rounded;
(ii) a water absorbing member arranged in the groove,
wherein a reinforcing film is provided on the surface of the water absorbing member, wherein the reinforcing film is in contact with the bottom part of the groove.

2. The tear meniscus test device according to claim 1, wherein the reinforcing film is colored.

3. The tear meniscus test device according to any one of claims 1 to 2. wherein a pigment is placed with a dot pattern in a part or the whole of the water absorbing member.

4. The tear meniscus test device according to any one of claims 1 to 2, wherein a pigment is placed continuously over a part or the whole of the water absorbing member.

5. The tear meniscus test device according to any one of claims 1 to 4, wherein the water absorbing member is formed of nitrocellulose.

6. The tear meniscus test device according to any one of claims 1 to 5, wherein a scale is marked on the body part along the groove.

7. A manufacturing method for a tear meniscus test device comprising the steps of:
- forming a body part sheet by forming a plurality of elongated slits by stamping a sheet formed of a synthetic resin or synthetic rubber and by bonding an adhesive film to one surface of the sheet;
- cutting a water absorbing member sheet into an elongated shape;
- arranging a water absorbing member, which is obtained by cutting the water absorbing member sheet into an elongated shape, in a groove formed by the slit and bonding the water absorbing member to the adhesive film forming the bottom part of the groove; and
- cutting the body part sheet into an elongated shape along the groove.

8. The manufacturing method for a tear meniscus test device according to claim 7, wherein one surface of the water absorbing member sheet is covered with a reinforcing film, and the water absorbing member cut into an elongated shape is embedded in the groove so that the reinforcing film is in contact with the adhesive film.

9. A manufacturing method for a tear meniscus test device comprising the steps of:
- forming an elongated body part sheet formed with a concave groove along the length direction thereof by extrusion molding or injection molding of synthetic resin or synthetic rubber;
- cutting a water absorbing member sheet provided with an adhesive film on one surface thereof into a plurality of elongated strips; and
- arranging a water absorbing member, which is obtained by cutting the water absorbing member sheet into an elongated shape, in the groove in the body part sheet and bonding the water absorbing member to the bottom part of the groove via the adhesive film.

10. The manufacturing method for a tear meniscus test device according to claim 9, wherein the one surface of the water absorbing member sheet is covered with a reinforcing film, and the adhesive film is bonded to the surface of the reinforcing film.

11. The manufacturing method for a tear meniscus test device according to claim 8 or 10, wherein the method comprises a step of coloring the reinforcing film.

## Patentansprüche

1. Eine Tränenmeniskus-Testvorrichtung umfassend:
(i) ein Rumpfteil, das eine langgestreckte Form aufweist, wobei
- das Rumpfteil ein synthetisches Harzmaterial oder ein synthetisches Gummimaterial umfasst;
- im Rumpfteil eine Aussparung in Längsrichtung des Rumpfteils gebildet ist; und
- das Rumpfteil flexibel ist und dessen vordere Frontpartie abgerundet ist;
(ii) ein wasserabsorbierendes Element, das in der Aussparung angeordnet ist, wobei ein Verstärkungsfilm auf der Oberfläche des wasserabsorbierenden Elements zur Verfügung gestellt wird, wobei der Verstärkungsfilm in Kontakt mit dem Unterteil der Aussparung steht.

2. Die Tränenmeniskus-Testvorrichtung gemäß Anspruch 1, wobei der Verstärkungsfilm gefärbt ist.

3. Die Tränenmeniskus-Testvorrichtung gemäß einem der Ansprüche 1 bis 2, wobei ein Pigment mit einem Punktmuster in einem Teil des wasserabsorbierenden Elements oder dem ganzen wasserabsorbierenden Element positioniert wird.

4. Die Tränenmeniskus-Testvorrichtung gemäß einem der Ansprüche 1 bis 2, wobei ein Pigment über einen Teil des wasserabsorbierenden Elements oder über das ganze wasserabsorbierende Element kontinuierlich positioniert wird.

5. Die Tränenmeniskus-Testvorrichtung gemäß einem der Ansprüche 1 bis 4, wobei das wasserabsorbierende Element aus Nitrocellulose gebildet wird.

6. Die Tränenmeniskus-Testvorrichtung gemäß einem der Ansprüche 1 bis 5, wobei eine Skala entlang der Aussparung auf dem Rumpfteil eingerichtet wird.

7. Ein Herstellungsverfahren für eine Tränenmeniskus-Testvorrichtung umfassend die Schritte aus:
- Bilden einer Lage aus Rumpfteilen durch Bilden einer Vielzahl von länglichen Schlitzen durch Stanzen einer Lage gebildet aus synthetischem Harz oder synthetischem Gummi und Binden eines Klebstofffilms an eine Oberfläche der Lage;
- Schneiden einer Lage aus wasserabsorbierenden Elementen in eine längliche Form;
- Anordnen eines wasserabsorbierenden Elements, das durch Schneiden der Lage aus wasserabsorbierenden Elementen in eine längliche Form erhalten wurde, in eine Aussparung, die durch den Schlitz gebildet wird, und Binden des wasserabsorbierenden Elements an den Klebstofffilm, der den Unterteil der Aussparung bildet; und
- Schneiden der Lage aus Rumpfteilen in eine längliche Form entlang der Aussparung.

8. Das Herstellungsverfahren für eine Tränenmeniskus-Testvorrichtung gemäß Anspruch 7, wobei eine Oberfläche der Lage aus wasserabsorbierenden Elementen mit einem Verstärkungsfilm bedeckt wird und das wasserabsorbierende Element, das in eine längliche Form geschnitten ist, in die Aussparung eingelassen wird, so dass der Verstärkungsfilm in Kontakt mit dem Klebstofffilm ist.

9. Ein Herstellungsverfahren für eine Tränenmeniskus-Testvorrichtung umfassend die Schritte aus:
- Bilden einer Lage aus länglichen Rumpfteilen mit einer konkave Aussparung in deren Längsrichtung durch Strangpressen oder Spritzguss eines synthetischen Harzes oder synthetischen Gummis;
- Schneiden einer Lage aus wasserabsorbierenden Elementen, die auf deren einer Seite einen Klebstofffilm aufweisen, in eine Vielzahl von länglichen Streifen; und
- Anordnen eines wasserabsorbierenden Elements, das durch Schneiden der Lage aus wasserabsorbierenden Elementen in eine längliche Form erhalten wurde, in die Aussparung in der Lage aus Rumpfteilen und Binden des wasserabsorbierenden Elements an das Unterteil der Aussparung durch einen Klebstofffilm.

10. Das Herstellungsverfahren für eine Tränenmeniskus-Testvorrichtung gemäß Anspruch 9, wobei die eine Oberfläche der Lage aus wasserabsorbierenden Elementen mit einem Verstärkungsfilm bedeckt wird und der Klebstofffilm an die Oberfläche des Verstärkungsfilms gebunden wird.

11. Das Herstellungsverfahren für eine Tränenmeniskus-Testvorrichtung gemäß Anspruch 8 oder 10, wobei das Verfahren einen Schritt zum Färben des Verstärkungsfilms umfasst.

## Revendications

1. Dispositif de test des ménisques de larmes, comprenant:
(i) une partie de corps présentant une forme allongée, dans lequel:
- la partie de corps contient une matière de résine synthétique ou une matière de caoutchouc synthétique;
- une rainure est formée dans la partie de corps le long d'un sens de la longueur de la partie de corps; et
- la partie de corps est souple et la partie d'extrémité avant de celle-ci est arrondie;
(ii) un élément d'absorption d'eau agencé dans la rainure,
dans lequel un film de renforcement est prévu sur la surface de l'élément d'absorption d'eau, dans lequel le film de renforcement est en contact avec la partie de fond de la rainure.

2. Dispositif de test des ménisques de larmes selon la revendication 1, dans lequel le film de renforcement est coloré.

3. Dispositif de test des ménisques de larmes selon l'une quelconque des revendications 1 à 2, dans lequel un pigment est placé avec un motif en points dans une partie ou dans la totalité de l'élément d'absorption d'eau.

4. Dispositif de test des ménisques de larmes selon l'une quelconque des revendications 1 à 2, dans lequel un pigment est placé de façon continue sur une partie ou sur la totalité de l'élément d'absorption d'eau.

5. Dispositif de test des ménisques de larmes selon l'une quelconque des revendications 1 à 4, dans lequel l'élément d'absorption d'eau est constitué de nitrocellulose.

6. Dispositif de test des ménisques de larmes selon l'une quelconque des revendications 1 à 5, dans lequel une échelle graduée est marquée sur la partie de corps le long de la rainure.

7. Procédé de fabrication d'un dispositif de test des ménisques de larmes comprenant les étapes suivantes:
- former une feuille de partie de corps en pratiquant une pluralité de fentes allongées en poinçonnant une feuille constituée d'une résine synthétique ou d'un caoutchouc synthétique et en collant un film adhésif sur une surface de la feuille;
- découper une feuille d'élément d'absorption d'eau selon une forme allongée;
- agencer un élément d'absorption d'eau, qui est obtenu en découpant la feuille d'élément d'absorption d'eau selon une forme allongée, dans une rainure formée par la fente et coller l'élément d'absorption d'eau sur le film adhésif qui constitue la partie de fond de la rainure; et
- découper la feuille de partie de corps selon une forme allongée le long de la rainure.

8. Procédé de fabrication d'un dispositif de test des ménisques de larmes selon la revendication 7, dans lequel une surface de la feuille d'élément d'absorption d'eau est recouverte d'un film de renforcement, et l'élément d'absorption d'eau découpé selon une forme allongée est encastré dans la rainure de telle sorte que le film de renforcement soit en contact avec le film adhésif.

9. Procédé de fabrication d'un dispositif de test des ménisques de larmes comprenant les étapes suivantes:
- former une feuille de partie de corps allongée pourvue d'une rainure concave le long du sens de la longueur de celle-ci par moulage par extrusion ou par moulage par injection d'une résine synthétique ou d'un caoutchouc synthétique;
- découper une feuille d'élément d'absorption d'eau pourvue d'un film adhésif sur une surface de celle-ci en une pluralité de bandes allongées; et
- agencer un élément d'absorption d'eau, qui est obtenu en découpant la feuille d'élément d'absorption d'eau selon une forme allongée, dans la rainure dans la feuille de partie de corps et coller l'élément d'absorption d'eau sur la partie de fond de la rainure par l'intermédiaire du film adhésif.

10. Procédé de fabrication d'un dispositif de test des ménisques de larmes selon la revendication 9, dans lequel la surface de la feuille d'élément d'absorption d'eau est recouverte d'un film de renforcement, et le film adhésif est collé sur la surface du film de renforcement.

11. Procédé de fabrication d'un dispositif de test des ménisques de larmes selon la revendication 8 ou 10, dans lequel le procédé comprend une étape de coloration du film de renforcement.
